## Europäisches Patentamt

## European Patent Office

## Office européen des brevets

(11) Veröffentlichungsnummer: **0 219 660**
**B1**

(12) # EUROPÄISCHE PATENTSCHRIFT

(45) Veröffentlichungstag der Patentschrift:
**29.11.89**

(51) Int. Cl.⁴: **A 61 K 6/10, C 08 L 83/04**

(21) Anmeldenummer: **86112057.4**

(22) Anmeldetag: **01.09.86**

(54) **Silicon-Abformmassen.**

(30) Priorität: **13.09.85 DE 3532687**

(43) Veröffentlichungstag der Anmeldung:
**29.04.87 Patentblatt 87/18**

(45) Bekanntmachung des Hinweises auf die Patenterteilung:
**29.11.89 Patentblatt 89/48**

(84) Benannte Vertragsstaaten:
**AT BE CH DE FR GB IT LI NL SE**

(56) Entgegenhaltungen:
EP-A- 0 043 501
EP-A- 0 152 887
EP-A- 0 158 141
EP-A- 0 166 107
US-A- 3 565 825

**Die Akte enthält technische Angaben, die nach dem Eingang der Anmeldung eingereicht wurden und die nicht in dieser Patentschrift enthalten sind.**

(73) Patentinhaber: **BAYER AG,
D-5090 Leverkusen 1 Bayerwerk (DE)**

(72) Erfinder: **Schwabe, Peter, Dr., Dudweiler Strasse 17,
D-5090 Leverkusen 1 (DE)**
Erfinder: **Voigt, Reiner, Dipl.-Ing.,
Gustav-Radbruch-Strasse 14, D-5090 Leverkusen 3 (DE)**
Erfinder: **Schlak, Ottfried, Dr.,
Carl-Duisberg-Strasse 331, D-5090 Leverkusen 1 (DE)**

**Beschreibung**

Die vorliegende Erfindung betrifft isoparaffinhaltige Massen auf Polysiloxanbasis, die als Abform- und/oder Dubliermassen, insbesondere im Dentalbereich Anwendung finden. Dabei handelt es sich um additionsvernetzende Siliconpasten zum Herstellen genauer Abformungen bezahnter, teilbezahnter und unbezahnter Kiefer sowie von Gipsmodellen im Dentalbereich.

Silicon-Pasten sind in der Zahnheilkunde als Abformmassen weit verbreitet. Im allgemeinen bestehen sie aus einem mit Füllstoffen vermischten Siliconöl auf Basis eines endständige Hydroxylgruppen aufweisenden Polydimethylsiloxans, welches je nach Applikationsmethode in verschiedenen Konsistenzen erhältlich ist, und einer flüssigen oder pastenförmigen Härtekomponente, die ein Metallsalz einer Monocarbonsäure als Katalysator und einen Kieselsäureester als Vernetzer enthält. (vgl. z.B. W. Noll, Chemie und Technologie der Silicone, Verlag Chemie, Weinheim, 2. Auflage 1964, S. 339-340).

Diese beiden Komponenten werden vor der Anwendung miteinander gemischt und vernetzen bei Raumtemperatur innerhalb von 2-5 Minuten infolge einer Polykondensationsreaktion. Hierbei entstehen neben dem vernetzten Silicongummi noch geringe Mengen Alkohol, die langsam aus dem Gummi heraus diffundieren und eine lineare Schrumpfung hervorrufen, was bei den Abformungen zu Ungenauigkeiten führt.

Wesentlich geringer ist die lineare Schrumpfung bei den erst seit wenigen Jahren bekannten Vinylsilicon-Abformmassen. Diese Massen bestehen aus zwei Pasten: einer Siliconöl, Füllstoff und Vernetzer enthaltenden Basispaste und einer Siliconöl, Füllstoff und Katalysator enthaltenden Katalysatorpaste.

Bei dem Siliconöl handelt es sich hierbei um ein endständiges Vinylgruppen aufweisendes Polydimethylsiloxan, der Vernetzer enthält die reaktiven SiH-Gruppen und der Katalysator besteht vorzugsweise aus einem Platinkomplex. Zu der größeren Dimensionsgenauigkeit der Abformung kommt bei diesem System noch die bessere Dosierbarkeit von Basis- und Katalysatorpaste infolge gleicher Pasten-Viskosität und dem abgestimmten Mischungsverhältnis von 1:1 der beiden Pasten hinzu, sowie die völlige Geschmack- und Geruchslosigkeit der Pasten. (vgl. z.B. R.C. Craig, Restorative Dental Materials, The C.V. Moosbe Comp., St. Louis, 1980 S. 195 ff.).

Aus der EP-A 0 043 501 sind unter Ausschluß von Wasser lagerfähige plastische Organopolysiloxanformmassen bekannt geworden, die hydroxyendgestoppte Polydiorganosiloxane enthalten. Die Aushärtungszeit beträgt jedoch 1 bis 3 Tage, was für die Anwendung als Dentalabformmasse in der Mundhöhle eines Patienten nicht in Frage kommen kann. Aus der EP-A 152 887 und EP-A 158 141 sind Dentalabformmassen auf Basis vinylendgestoppter Organopolysiloxane bekannt geworden, die n-Paraffine enthalten, um die Klebrigkeit der Massen an den Händen des Verarbeiters zu reduzieren. Jedoch schwitzen sowohl derartige Pasten als auch die daraus hergestellten Abformungen während der Lagerung einen Teil des n-Paraffins aus, wodurch Abformungen und Modelle mit nicht ausreichender Präzision erhalten werden.

Im Rahmen der Bemühungen, derartige Abformmassen weiter zu verbessern und zu modifizieren wurde überraschenderweise gefunden, daß Isoparaffine mit 8-24 C-Atomen sich mit solchen Massen mischen lassen und daß dabei hochwertige Abformmassen erzielt werden können.

Gegenstand der Erfindung ist somit die Verwendung von Isoparaffinen mit 8-24 C-Atomen für pastenförmige Siliconmassen, im Dentalbereich wobei die erfindungsgemäßen Isoparaffine vorzugsweise in einer Menge von 1-50 Gew.-%, insbesondere 5-30 Gew.-%, angewandt werden und vorzugsweise Isohexadecan und Isoeikosan sind.

Gegenstand der Erfindung sind darüber hinaus auch Siliconpasten, insbesondere Silicon-Abform- und Dubliermassen, insbesondere für den Dentalbereich, welche derartige Isoparaffine enthalten.

Vorzugsweise werden die erfindungsgemäßen Isoparaffine in bei Raumtemperatur härtbaren Abformmassen auf Polysiloxanbasis verwendet. Man unterscheidet dabei, wie schon eingangs ausgeführt, additionsvernetzende und kondensationsvernetzende Systeme. Als wesentliche Komponenten enthalten nach dem Additionssystem vernetzende erfindungsgemäße Pasten

    a) Organopolysiloxane mit zwei oder mehr Vinylgruppen im Molekül,

    b) anorganische Füllstoffe, (unbehandelt oder oberflächenmodifiziert),

    c) Organohydrogenpolysiloxane als Vernetzer,

    d) einen Katalysator zur Beschleunigung der Additionsreaktion,

    e) Isoparaffin

    f) und gegebenenfalls übliche Zusatzstoffe (wie z.B. Geschmacks- und Geruchsstoffe).

Die erfindungsgemäßen Siliconmassen zeichnen sich durch ihre Lagerstabilität und Gleitfähigkeit beim Vermischen von Basis- und Katalysatorpaste bzw. von Paste und Härterkomponente aus. Sie eignen sich für die Herstellung genauer Abformungen, speziell auch von Zähnen wegen ihrer detailgetreuen Wiedergabe im Gipsmodell, nachdem die Vernetzer- und Katalysatorpaste gut gemischt in die Mundhöhle eingebracht und darin verfestigt wurde, die Abformung mit einer Gipsaufschlämmung ausgegossen und zu einem Modell ausgehärtet worden ist. Dieses gute Ergebnis wird erzielt, weil durch das erfindungsgemäß eingesetzte Isoparaffin das Benetzungsverhalten sowohl der angemischten Silicon-Abformmasse auf der immer feuchten zahn- und Schleimhautoberfläche, als auch das der vernetzten Abformung gegenüber der Gipsaufschlämmung verbessert wurde.

Die additionsvernetzenden Dubliermassen zeichnen sich durch niedrige Viskosität des als Verdünnungsmittel erfindungsgemäß eingesetzten Isoparaffin und genaue Abbildungsschärfe, auch durch das verbesserte Benetzungsverhalten der Masse gegenüber dem abzubildenden Gipsmodells aus sowie durch Flexibilität der vernetzten Dublierform. Letztere läßt sich wiederum durch das verbesserte Benetzungsverhalten mit einer Gipsaufschlämmung gut ausgießen.

Die Einsatzstoffe der oben genannten, bei Raumtemperatur härtbaren Pasten sind an sich bekannt.

Bei dem Siliconöl (a) handelt es sich um ein vinylendgestopptes Polydimethylsiloxan, dessen Viskosität bevorzugt im Bereich von 500 bis 5 000 000 mPa·s bei 20°C liegt.

Als Füllstoffe (b) eignen sich z.B. Quarz- und Cristobalitmehle, Calciumsulfat, Diatomeenerde, Talkum und Calciumcarbonat sowie gefälltes und pyrogen hergestelltes Siliciumdioxid mit unbehandelter und modifizierter Oberfläche.

Der Vernetzer (c) ist ein Polydimethylsiloxan, welches in seinem Molekül Wasserstoffatome an mindestens zwei Siliciumatomen aufweist.

Bei dem Katalysator (d) handelt es sich z.B. um einen Platinkomplex, der aus Hexachlorplatin-(IV)-säure hergestellt wurde. Auch diese Verbindungen sind an sich bekannt.

Die erfindungsgemäß zu verwendenden Isoparaffine enthalten 8-24 Kohlenstoffatome im Molekül, bevorzugt werden Isohexadecan $C_{16}H_{34}$ und Isoeikosan $C_{20}H_{42}$.

Wie eingangs geschildert, werden die additionsvernetzenden Vinylsiliconabformmassen in verschiedenen Konsistenzen jeweils als 2-Pasten-System angeboten, einer Siliconpolymer, Vernetzer und Füllstoff enthaltenden Basispaste und einer Katalysatorpaste aus Siliconpolymer, Füllstoff und Katalysator.

Die beiden Pasten, vorzugsweise 1:1 gewichts- und/oder volumenmäßig dosiert, werden auf einem Anmischblock mittels Spatel homogen vermischt, auf einen Abformlöffel plaziert und auf die abzuformende Kieferpartie gedrückt. Nach 2-5 Minuten kann die inzwischen zu Gummi vernetzte Abformung aus dem Mund genommen werden. Das erhaltene Negativ der Kiefersituation zeichnet sich durch eine genaue Zeichnung aus, da die eingesetzte Masse mit dem erfindungsgemäßen Isoparaffin ein verbessertes Benetzungsverhalten gegenüber den immer feuchten Zahn- und Schleimhautoberflächen aufweist, als Vinylsilicon-Abformmassen ohne Isoparaffin. Das verbesserte Benetzungsverhalten zeigt sich auch beim anschließenden Ausgießen der Abformung mit einer Gipsaufschlämmung. Neben diesen Eigenschaften ist das erfindungsgemäße Isoparaffin eine kostensenkende Flüssigkomponente in den Pastenrezepturen.

Zur Vervielfältigung von Gipsmodellen werden niedrigviskose additionsvernetzende Dubliermassen eingesetzt, bestehend aus einer Vinylsiliconöl, Vernetzer und Füllstoff enthaltenden Basispaste und einer Katalysatorpaste aus Vinylsiliconöl, Füllstoff und Katalysator. Die beiden Pasten werden in einem Becher gewichts- und/oder volumenmäßig 1:1 bis vorzugsweise 9:1 gemischt und die Mischung in eine das zu vervielfältigende Gipsmodell enthaltende Küvette gegossen. Nach 10-25 Minuten ist die Masse vernetzt und nach dem Ausformen besitzt man die Form zur Herstellung weiterer Gipsmodelle. Beim Einsatz der erfindungsgemäßen Isoparaffine konnte neben den oben genannten Vorteilen wie Benetzungsverbesserung und Kostensenkung festgestellt werden, daß die vernetzten Dubliermassen viel weniger beim Mischen und Eingießen entstandene Luftbläschen enthielten und viel flexibler als bisherige Dublierformen waren.

Abschließend konnte das verbesserte Benetzungsverhalten durch das erfindungsgemäß eingesetzte Isoparaffin bei der Herstellung der Pasten beim Vermischen von Siliconölen mit Füllstoffen festgestellt werden.

Die nachfolgenden Beispiele, in denen alle Teile Gewichtsteile bedeuten, erläutern die Erfindung.

*Beispiel 1* (Vergleich)

Die Basispaste einer mittelviskosen additionsvernetzenden Dental-Abformmasse wurde hergestellt durch Vermischen von einem Kneter von 340 Teilen vinylendgestopptem Polydimethylsiloxan mit einer Viskosität von 10 000 mPa·s bei 20°C, 210 Teilen dimethylhydrogensiloxy-endgestopptem Polydimethylsiloxan mit einer Viskosität von 120 mPa·s bei 20°C, 380 Teilen Quarzfeinstmehl, 50 Teilen pyrogen hergestellter Kieselsäure mit einer spezifischen Oberfläche von 50 m²/g nach BET und 20 Teilen anorganischem Farbstoff.

Die Herstellung der Katalysatorpaste erfolgte durch Vermischen in einem Kneter von 548 Teilen vinylendgestopptem Polydimethylsiloxan mit einer Viskosität von 5 000 mPa·s bei 20°C, 400 Teilen Quarzfeinstmehl, 50 Teilen der oben genannten Kieselsäure, 1,8 Teilen Titandioxid und 0,2 Teilen eines Komplexes aus Platin und Divinyltetramethyldisiloxan.

Vor der Anwendung werden Basispaste und Katalysatorpaste gewichtsmäßig 1:1 gemischt. Die Massen entsprachen der Spezifikation DIN 13 913 und der Spezifikation 19 der American Dental Association. Wassertropfen auf dem ausgehärteten Gummi wiesen einen Benetzungswinkel von 122° auf.

*Beispiel 2* (erfindungsgemäß)

In einem Kneter wurde eine Basispaste hergestellt durch Vermischen von 220 Teilen vinylendgestopptem Polydimethylsiloxan mit einer Viskosität von 10 000 mPa·s bei 20°C, 140 Teilen dimethylhydrogensiloxy-endgestopptem Polydimethylsiloxan aus Beispiel 1, 150 Teilen Isoeikosan, 410 Teilen Quarzfeinstmehl, 60 Teilen Kieselsäure aus Beispiel 1 und 20 Teilen anorganischem Farbstoff.

Die Katalysatorpaste wurde hergestellt in einem Kneter durch Vermischen von 368 Teilen vinylendgestopptem Polydimethylsiloxan mit einer Viskosität von 5 000 mPa·s bei 20°C, 150 Teilen Isoeikosan, 420 Teilen Quarzfeinstmehl, 60 Teilen Kieselsäure aus Beispiel 1, 1,8 Teilen Titandioxid und 0,2 Teilen Platinkomplex aus Beispiel 1.

Die Basis- und Katalysatorpaste werden vor Anwendung gewichtsmäßig 1:1 gemischt. Die Massen entsprechen den in Beispiel 1 angegebenen Spezifikationen. Der Benetzungswinkel der Wassertropfen auf dem ausgehärteten Gummi betrug 96°.

*Beispiel 3* (Vergleich)

Die Basispaste einer additionsvernetzenden Dubliermasse wurde hergestellt in einem Kneter durch Vermischen von 550 Teilen vinylendgestopptem Polydimethylsiloxan mit einer Viskosität von 10 000 mPa·s bei 20°C, 200 Teilen trimethylsiloxy-endgestopptem Polydimethylsiloxan mit einer Viskosität von 120 mPa·s bei 20°C, 200 Teilen SiH-gruppenhaltigem Polydimethylsiloxan mit einer Viskosität von 95 mPa·s bei 20°C und 50 Teilen einer gefällten und oberflächenbehandelten Kieselsäure mit einer Oberfläche von 90 m²/g nach BET.

In einem Kneter wurde die Katalysatorpaste hergestellt durch Vermischen von 540 Teilen vinylendgestoppten Polydimethylsiloxan mit einer Viskosität von 10 000 mPa·s bei 20°C, 400 Teilen trimethylsiloxy-endgestopptem Polydimethylsiloxan mit einer Viskosität von 120 mPa·s bei 20°C, 50 Teilen oben angegebener Kieselsäure, 9,8 Teilen anorganischem Farbstoff und 0,2 Teile Platin-Siloxan-Komplex aus Beispiel 1.

Die Basispaste wurde vor der Anwendung mit der Katalysatorpaste im Gewichtsverhältnis 9:1 gemischt. Die ausgehärtete Dublierform enthielt vom Mischen und Eingießen entstandene Luftblasen. Der Benetzungswinkel der Wassertropfen auf dem vernetzten Gummi betrug 125°.

*Beispiel 4* (erfindungsgemäß)

Die Herstellung der Basispaste erfolgte in einem Kneter durch Mischen von 500 Teilen vinylendgestopptem Polydimethylsiloxan aus Beispiel 5, 250 Teilen Isoeikosan, 180 Teilen SiH-gruppenhaltigem Polydimethylsiloxan aus Beispiel 5, 70 Teilen Kieselsäure aus Beispiel 5.

Die Katalysatorpaste wurde in einem Kneter hergestellt durch Mischen von 480 Teilen vinylendgestopptem Polydimethylsiloxan aus Beispiel 5, 450 Teilen Isoeikosan, 60 Teilen Kieselsäure aus Beispiel 5, 9,8 Teilen anorganischem Farbstoff und 0,2 Teile Platin-Siloxan-Komplex aus Beispiel 1.

Die Basis- und Katalysatorpasten wurden vor der Anwendung im Gewichtsverhältnis 9:1 gemischt. Sehr wenige, vom Mischen und Eingießen entstandene Luftblasen wiesen die vernetzende Dublierform auf. Wassertropfen auf dem vernetzende Gummi wiesen einen Benetzungswinkel von 85° auf.

## Patentansprüche

1. Additionsvernetzende Abform- bzw. Dubliermassen, insbesondere für den Dentalbereich auf Silikonbasis bestehend aus
A) einer Basiskomponente bestehend aus
a) Organopolysiloxanen mit 2 oder mehr Vinylgruppen im Molekül
b) Organohydrogenpolysiloxanen als Vernetzer
c) unbehandelten oder oberflächenmodifizierten anorganischen Füllstoffen
und
B) einer Katalysatorkomponente bestehend aus
a) Organopolysiloxanen mit 2 oder mehr Vinylgruppen im Molekül
b) einem Katalysator auf Platinbasis
c) unbehandelten oder oberflächenmodifizierten anorganischen Füllstoffen,
dadurch gekennzeichnet, daß die Basiskomponente A) und/oder die Katalysatorkomponente B) Isoparaffine mit 8 bis 24 C-Atomen enthalten.

2. Massen gemäß Anspruch 1, dadurch gekennzeichnet, daß als Isoparaffine Isohexadecan und/oder Isoeikosan eingesetzt werden.

3. Verwendung von Massen gemäß einem der Ansprüche 1 oder 2 zur Herstellung von Abform- bzw. Dubliermassen für den Dentalbereich.

## Claims

1. Addition-crosslinking impression compounds or duplication compounds, in particular for the dental field, based on silicones and consisting of
A) a base component consisting of
a) organopolysiloxanes having 2 or more vinyl groups in the molecule,
b) organohydrogenpolysiloxanes as crosslinking agents and
c) untreated or surface-modified inorganic fillers and
B) a catalyst component consisting of
a) organopolysiloxanes having 2 or more vinyl groups in the molecule,
b) a platinum-based catalyst and
c) untreated or surface-modified inorganic fillers, characterized in that the base component A) and/or the catalyst component B) contain isoparaffins having 8 to 24 C atoms.

2. Compounds according to Claim 1, characterized in that isohexadecane and/or isoeicosane are used as the isoparaffins.

3. Use of compounds according to one of Claims 1 or 2 for the preparation of impression compounds or duplication compounds for the dental field.

## Revendications

1. Masses de moulage ou de reproduction réticulant par addition, conçues en particulier pour l'art dentaire, à base de silicones, qui consistent en
A) un composant de base consistant lui-même en
a) des organopolysiloxannes contenant deux groupes vinyle ou plus par molécule,
b) des organohydrogénopolysiloxannes qui servent d'agents réticulants,
c) des matières de charge minérales non traitées ou modifiées en surface,
et
B) un composant catalyseur consistant en
a) des organopolysiloxannes contenant deux groupes vinyle ou plus par molécule,
b) un catalyseur à base de platine,
c) des matières de charge minérales non traitées ou modifiées en surface,
caractérisées en ce que le composant de base A) et/ou le composant catalyseur B) contiennent des isoparaffines $C_8$-$C_{24}$.

2. Masses selon la revendication 1, caractérisées en ce que les isoparaffines utilisées sont l'isohexadécane et/ou l'isoéicosane.

3. Utilisation des masses selon l'une des revendications 1 ou 2 pour la préparation de masses de moulage ou de reproduction pour la dentisterie.